# EUROPEAN PATENT APPLICATION

(11) **EP 4 390 397 A1**
(43) Date of publication of application: **26.06.2024**
(21) Application number: 22383270.0
(22) Date of filing: 23.12.2022
(51) Int. Cl.: G01N 33/576, C12Q 1/6883, G01N 33/68, G01N 33/574

(54) **METHODS FOR DIAGNOSING LIVER DISEASE**

(71) Applicant: Asociación Centro de Investigación Cooperativa en Biociencias - CIC bioGUNE, 48160 Derio, Bizkaia (Bizkaia) (ES); Fundación Tekniker, 20600 Eibar (Guipuzcoa) (ES); Fundación Gaiker, 48170 Zamudio, Bizkaia (ES)
(72) Inventor: MARTÍNEZ CHANTAR, María Luz, 48160 Derio, Bizkaia (ES); MABE ÁLVAREZ, Jon, 20600 Eibar, Guipúzcoa (ES); RODRÍGUEZ AGUDO, Rubén, 48160 Derio, Bizkaia (ES); BERGANZA GRANDA, Jesús, 48170 Zamudio, Bizkaia (ES); GOÑI DE CERIO, Felipe, 48170 Zamudio, Bizkaia (ES)
(74) Representative: ABG Intellectual Property Law, S.L.

(57) **Abstract**

The present invention relates to a method for diagnosing liver disease in a subject and/or for triaging a subject for liver disease, which is based on the detection of the expression level of GNMT in a urine sample from said subject. The invention also relates to methods for monitoring progression of liver disease, for monitoring a patient's response to a therapy for liver disease, and for selecting a subject to be treated for liver disease. The invention also describes the use of reagents specific for determining the expression level of GNMT in a urine sample from a subject, for *in vitro* diagnosing a liver disease in said subject.

## Description

### TECHNICAL FIELD OF THE INVENTION

The present invention relates to the field of triaging, diagnosing and/or monitoring an acute or a chronic liver disease. More particularly, the invention relates to the use of GNMT as a marker for a hepatic disease.

### BACKGROUND OF THE INVENTION

The liver is the largest solid and most metabolically complex organ in the human body. It is involved in major vital functions such as digestion, detoxification, metabolism, immunity and nutrient storage and performs many essential biological processes including glucose homeostasis, synthesis of fatty acids (FAs), amino acids and blood proteins, production of bile and clearance of the blood from harmful substances. All these functions make the liver an essential organ providing energy and nutrients to the rest of the body. However, due to its central role in metabolism, the liver is exposed to a large amount of toxicant and is more susceptible to injury and dysfunction that other organs. It is an organ with capacity for natural regeneration.

Although liver disease is stereotypically linked to alcohol or viral infections, there are over 100 forms of liver disease caused by a variety of factors affecting everyone from infants to older adults. Furthermore, liver diseases can be acute with fulminant liver failure or chronic and evolve over time to cirrhosis and liver cancer. Genetic and autoimmune alterations are important causes of cholestatic liver injury and liver failure that mostly affect children. Also, changes in the way of life like bad dietary habits (obesity and diabetes) leading to Non-Alcoholic Fatty Liver Disease (NAFLD) that encompass a spectrum of abnormalities that range from steatosis, NASH, fibrosis and liver cancer as well as an excessive drug consumption have become major risk factors for liver disease. Indeed, the prevalence and mortality rates of liver disease have been increasing for the past 20 years and represent a global health problem. According to different estimates 1,7 billon of people suffer NAFLD, 29 million people in Europe suffer from chronic liver injury, 35 million in the United States have liver disease and about 2 million patients die annually from cirrhosis and liver cancer.

During their routinely analysis, clinicians use biopsy and biochemical tests as gold-standard for the liver study. Liver biopsies are invasive, carrying a risk of infection, and commonly not comprehensive of the entire organ or tissue. Besides those tests, the daily clinical practice uses expensive and sometimes inaccurate imaging resolution methods, such as ultrasound, computed tomography (CT) and magnetic resonance imaging (MRI) (among others), that may be used to assess tissue health, but generally can only detect overt features and changes. Liver function tests, like bilirubin, albumin or blood platelet count, help to detect inflammation and liver damage. Hepatocytes death triggers inflammation causing increase in serum aminotransferases activity due to release of ALT and AST. ALT is specific of the hepatocytes and AST is found also in different organs for instance heart and muscle. Hence, these tests are generally not sensitive enough to pick up early onset of conditions or fairly recent developments of conditions.

Reliable and non-invasive early markers for diagnosing and patient monitoring are not available in liver clinical practice. As a consequence, new biomarkers should be developed in this healthcare area. The growing burden of global liver disease, the absence of symptoms until late in the natural history of a disease which may take decades to manifest, the presence of an invasive reference test (liver biopsy) to assess disease severity, and the lack of robust tools to assess the efficacy of therapeutic interventions are some of the key drivers for this research.

Currently there is a need for routinely non-invasive methods to detect and follow up liver damage. The only available procedures until date are invasive and frequently need a professional to perform those procedures, so developing a non-invasive alternative is needed. Besides, the rapid development of new medications for the treatment of some liver diseases, (Chronic Hepatitis B or CHB, Chronic Hepatitis C or CHC, NAFLD), increases the need for more frequent evaluation of liver damage to assess treatment response.

### SUMMARY OF THE INVENTION

The inventors have surprisingly found that elevated urine levels of GNMT can be used as an early onset biomarker for diagnosing several types of liver disease, even before an increase in serum aminotransferases activity due to release of ALT and AST can be detected.

Thus, in a first aspect, the invention refers to an *in vitro* method for diagnosing liver disease in a subject and/or for triaging a subject for liver disease, wherein the method comprises:
a) determining the expression level of GNMT in a urine sample from said subject, and
b) comparing said expression level of GNMT with a reference value,
wherein an increased expression level of GNMT in said urine sample with respect to the reference value is indicative that the subject suffers from liver disease.

In a further aspect, the invention refers to an *in vitro* method for monitoring progression of liver disease in a subject, wherein the method comprises:
a) determining the expression level of GNMT in a urine sample from said subject at a first period of time;
b) determining the expression level of GNMT in a urine sample from said subject at a second period of time, wherein said second period of time is later than said first period of time; and
c) comparing the expression level of GNMT at the second period of time with the expression level of GNMT at the first period of time,
wherein an increased expression level of GNMT at the second period of time with respect to the expression level of GNMT at the first period of time is indicative that the liver disease is progressing in the subject; wherein a decreased or an equal expression level of GNMT at the second period of time with respect to the expression level of GNMT at the first period of time is indicative that the liver disease is not progressing in the subject.

In yet a further aspect, the invention relates to an *in vitro* method for monitoring the response to a therapy in a subject suffering from liver disease, wherein the method comprises:
a) determining the expression level of GNMT in a urine sample from said subject prior to the administration of said therapy;
b) determining the expression level of GNMT in a urine sample from said subject during or after the administration of said therapy; and
c) comparing the expression level of GNMT during or after the administration of said therapy with the expression level of GNMT prior to the administration of said therapy,
wherein a decreased expression level of GNMT during or after the administration of said therapy with respect to the expression level of GNMT prior to the administration of said therapy is indicative that the subject has a positive response to the therapy; wherein an increased or an equal expression level of GNMT during or after the administration of said therapy with respect to the expression level of GNMT prior to the administration of said therapy is indicative that the subject does not have a positive response to the therapy.

In an additional aspect, the invention refers to a method for selecting a subject to be treated for liver disease, wherein the method comprises:
a) determining the expression level of GNMT in a urine sample from said subject, and
b) comparing said expression level of GNMT with a reference value,
wherein an increased expression level of GNMT in said urine sample with respect to the reference value is indicative that the subject is selected for treatment of liver disease.

In another additional aspect, the invention refers to the use of reagents specific for determining the expression level of GNMT in a urine sample from a subject, for *in vitro* diagnosing liver disease in said subject.

### BRIEF DESCRIPTION OF THE FIGURES

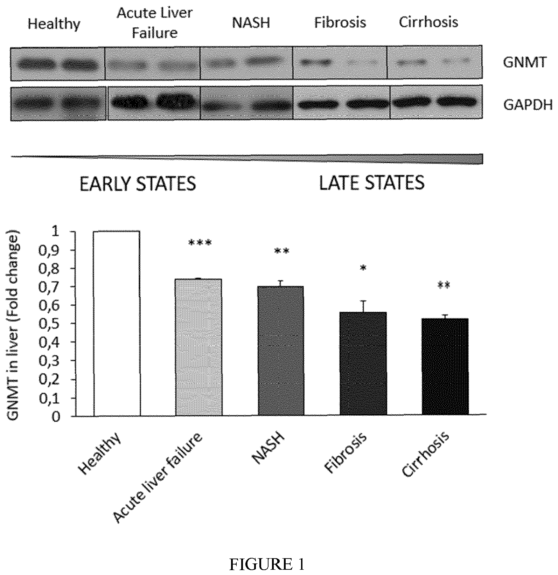

**Figure 1****.** GNMT determination by Western Blotting in liver extracts from different pathologies, both chronic liver disease (Non Alcoholic Steatohepatitis or NASH and fibrosis/cirrhosis) and acute liver failure (DILI). NASH model promoted by 0,1% methionine and choline-deficient diet (2 and 3 weeks), Fibrosis and fibrosis-cirrhosis genetic models were observed in genetically modified mice models Mdr2 -/- (at 3, early stages, and 5 months, advanced stages). Acute liver failure was provoked by acetaminophen induced acute liver injury with 300 mg of APAP per kg in 48 hours. Western Blotting on the upper part, fold change determination on the lower part. Quantification was normalized using GAPDH as a constitutive protein. GNMT downregulation is observed in all. *p<0.05 vs Healthy, **p<0.01 vs Healthy and ***p<0.001 vs Healthy.

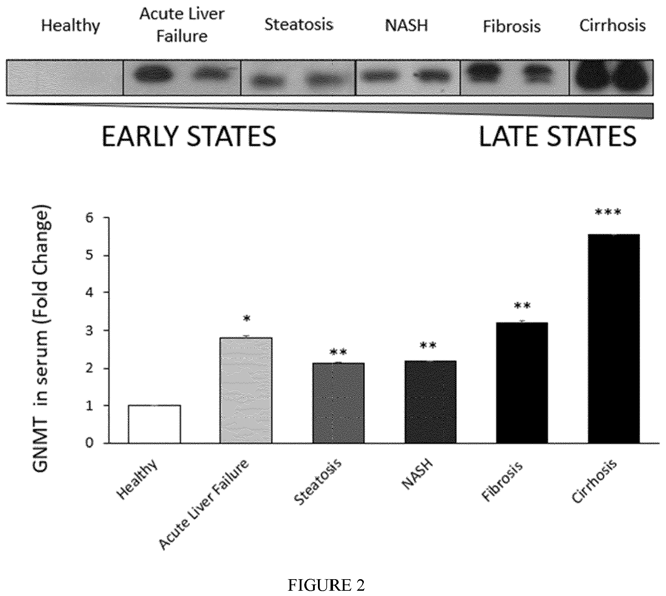

**Figure 2****.** A GNMT detection by Western Blotting in serum samples of different liver pathologies. Serum analysis was standardized to 1µl per sample. Here, the inventors analysed chronic liver disease (Non Alcoholic Steatohepatitis or NASH and fibrosis/cirrhosis) and acute liver failure (DILI). NAFLD and NASH models were promoted by 0,1% methionine and choline-deficient diet (at 2 and 4 weeks), fibrosis-cirrhosis genetic models were observed in Mdr2 -/- genetically modified mice models at 3 and 5 months of disease development. Acute liver failure was provoked by acetaminophen induced acute liver injury with 300 mg of APAP per kg in 48 hours. The downregulation observed in liver extract is accompanied by an increase of the marker in serum, absent in the GNMT-/- serum sample. Then, it shows GNMT fold change in serum by Western Blotting different pathologies. *p<0.05 vs Healthy, **p<0.01 vs Healthy and ***p<0.001 vs Healthy.

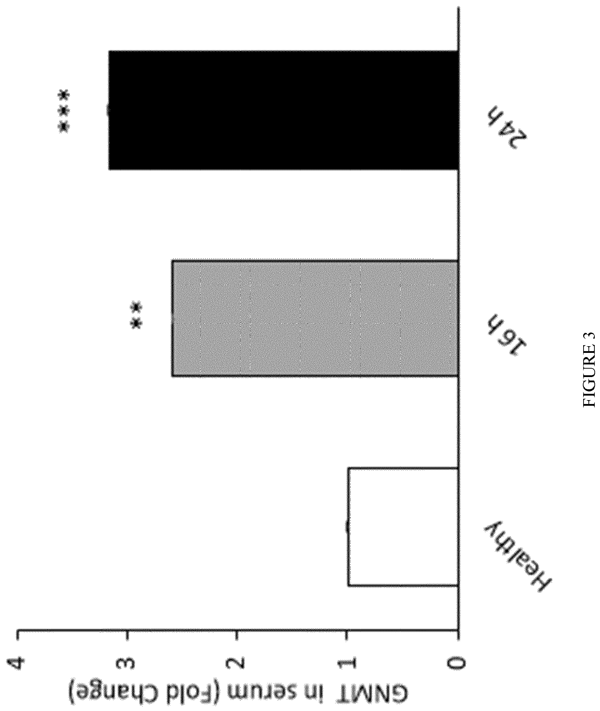

**Figure 3****.** Quantification of GNMT in serum by Western Blot at early stages of liver damage with starvation. Serum analysis was standardized to 1µl per sample. The downregulation of GNMT in liver is accompanied by an increase of the marker in serum. *p<0.05 vs Healthy and **p<0.01 vs Healthy.

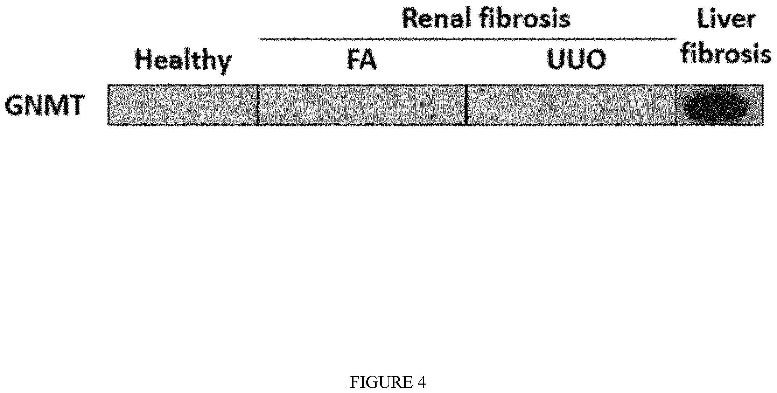

**Figure 4****.** GNMT determination by Western Blotting in serum samples. Serum analysis was standardized to 1µl per sample. Animal models of renal fibrosis (FA and UUO) were compared with healthy mice and animal models with liver fibrosis. GNMT in serum is only detected in liver pathologies, while no signal is observed in renal fibrosis models.

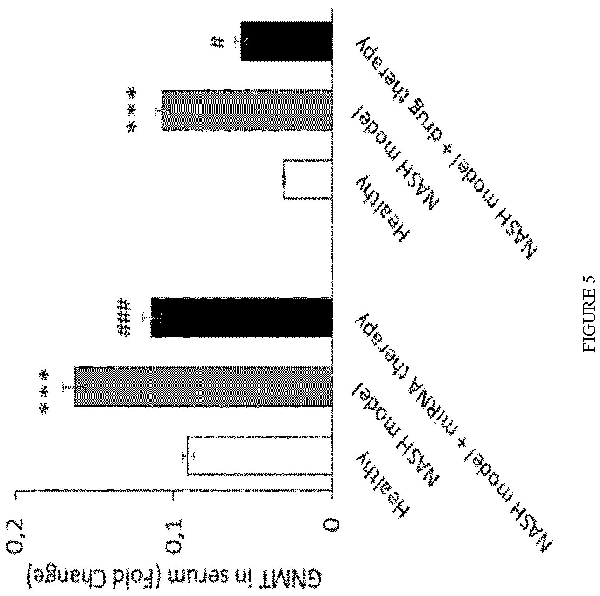

**Figure 5****.** GNMT determination by Western Blotting in serum samples. Serum analysis was standardized to 1µl per sample. The reversion in NASH pathological model was notably as a consequence of MLN4924 or anti-miR-873-5p therapies. The increase observed in the liver pathology condition gets partially reverted because of the treatment. ***p<0.001 vs Healthy, #p<0.05 vs NASH model and ###p<0.001 vs Fibrosis model.

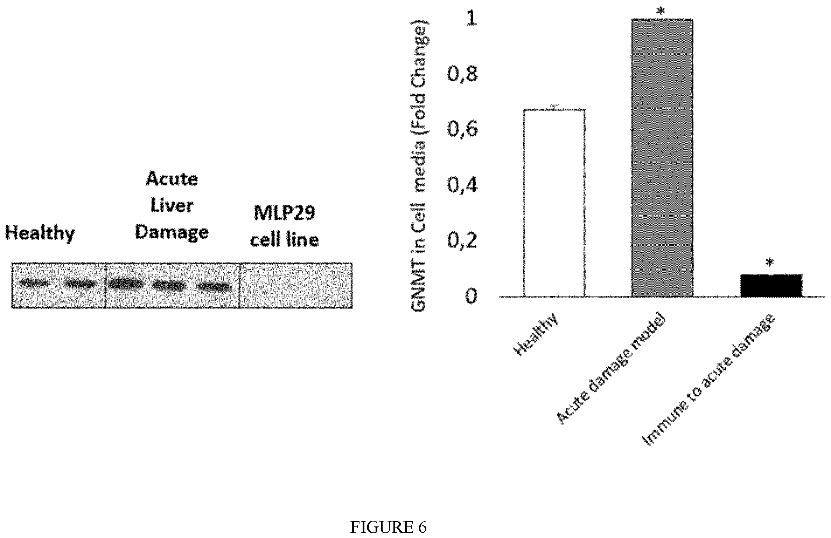

**Figure 6****.** GNMT is detected by Western Blotting in culture media of different cell types, healthy primary hepatocytes, hepatocytes with induced liver damage (acute damage) and mouse liver progenitor cells (MLP29). Also a fold change of GNMT expression in culture medium was measured. *p<0.05 vs Healthy and ***p<0.001 vs Healthy.

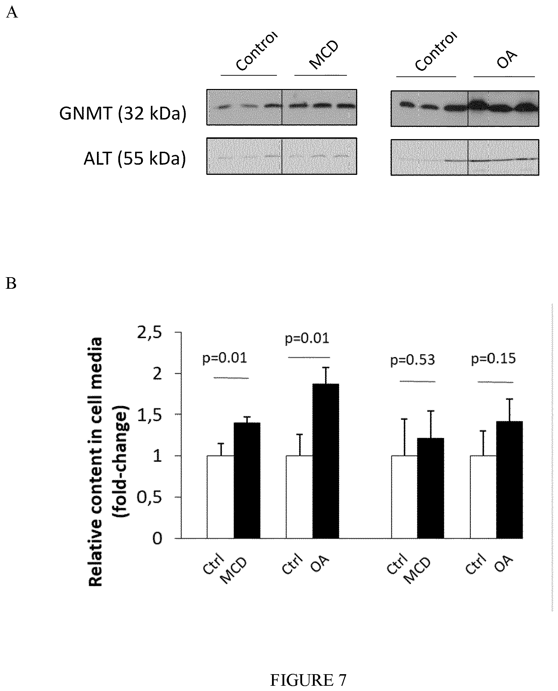

**Figure 7****.** In vitro glycine N-methyltransferase (GNMT) and alanine aminotransferase (ALT) determination in culture medium of mice primary hepatocytes under steatogenic stimuli. A. Western Blot analysis and B. relative quantification of GNMT and ALT proteins in the culture media of primary hepatocytes under 24h treatment of methionine and choline-deficient medium (MCD) or oleic acid (OA) to induce liver injury for toxic lipid accumulation. GNMT is detected in the culture medium as a marker for liver injury while ALT does not correlate with the hepatocyte injury.

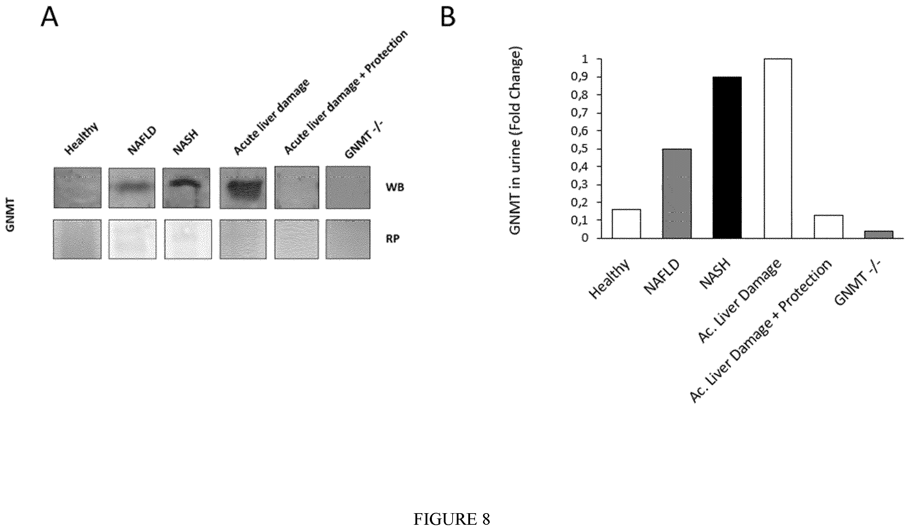

**Figure 8****.** GNMT detection by Western Blotting in different urine samples (30 µl per sample). Animal models were as follows: sample with acute liver damage (30 minutes after 70% partial hepatectomy), genetic model protection against damage, chronic liver damage (steatosis (2 weeks) and NASH (4 weeks), triggered by 0,1% methionine and choline-deficient diet) and GNMT-deficient mice (GNMT -/-). A) Western Blotting (WB) and red ponceau (RP) of the same wells. B) GNMT fold change quantification by densitometry. To sum up, GNMT is considered as a non-invasive routinely biomarker able to be used in very early stages of the liver damage. Hepatic protection through the modulation of a complex I mitochondrial inhibitor.

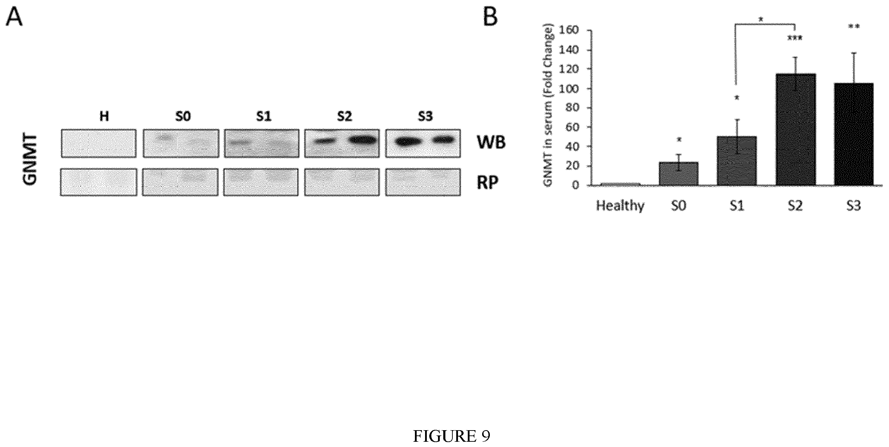

**Figure 9.** A) GNMT detection by Western Blot in human serum samples. Patients were defined by biopsy in different categories of steatosis. B) Quantification of detected GNMT by Western Blot in 31 human samples shows an increase GNMT directly related with the steatotic stage. A) shows Western Blotting and red ponceau (RP) as loading control. B) WB quantification by densitometry. *p<0.05 vs Healthy, **p<0.01 vs Healthy, ***p<0.001 vs Healthy and #<0.05 vs S1.

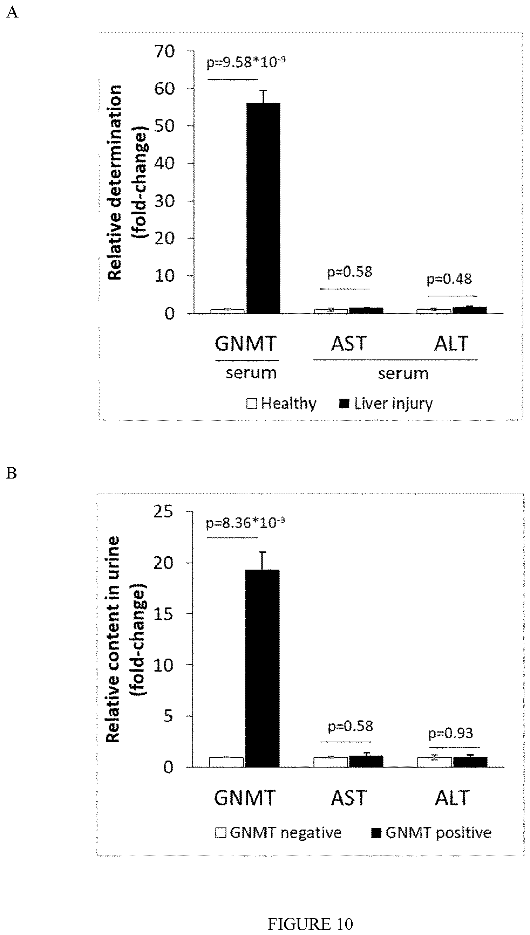

**Figure 10****.** Comparison of the different liver markers in body fluids. (A) The presence of glycine N-methyltransferase (GNMT) was determined by Western Blotting in 52 serum samples belong to liver patients with different etiologies and compared to the clinical parameters aspartate aminotransferase (AST) and alanine aminotransferase (ALT). GNMT appears in the serum of 44/44 patients with liver pathology versus 2/8 healthy samples. ALT and AST are utterly absent in the samples belong to liver pathologies conditions. (B) The activity of the aminotransferases aspartate aminotransferase (AST) and alanine aminotransferase (ALT) was measured in urine samples derived from patients with a GNMT-positive liver pathology. Importantly, in the samples belonging to these patients AST and ALT activities were absent.

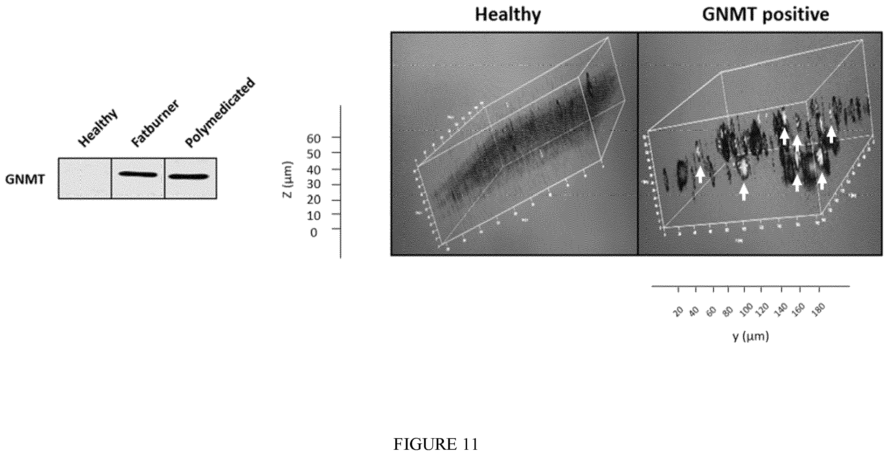

**Figure 11****.** A) GNMT detection by Western Blot in different urine samples from a human cohort. The biomarker can only be detected in patients with liver damage. B) Confocal microscopy pictures of a GNMT-positive sample compared to a healthy sample confirms the specificity of the Western Blot technique. The pale grey molecules (pointed arrows) correspond with GNMT; phospholipids were stained by Vybrant DIL (dark molecules); 63X3D.

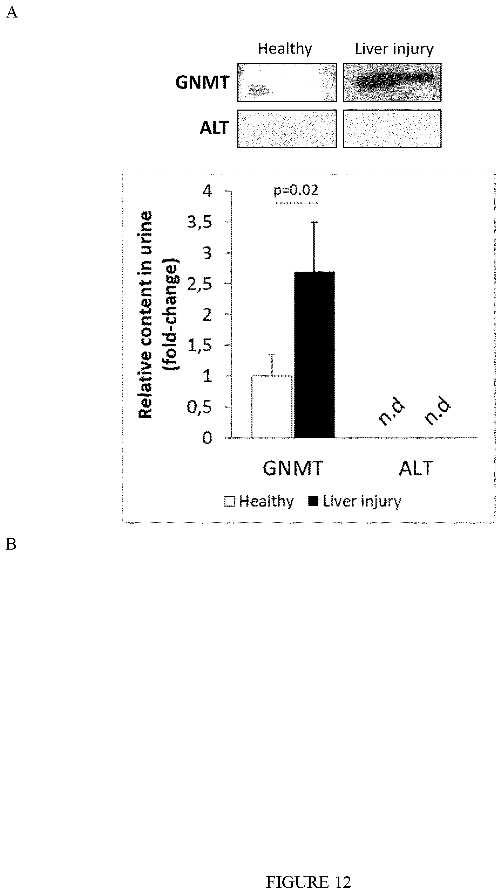

**Figure 12****.** The presence of glycine N-methyltransferase (GNMT) is increased in patients with liver injury and correlated with the fibrosis index. A) GNMT was determined by Western Blotting in a pre-validation of 40 urine sample belong to patients with liver pathology. The presence of the liver marker alanine aminotransferase (ALT) was not detected. B) The presence of GNMT appears to correlate with the grade of fibrosis, measured by Fibroscan.

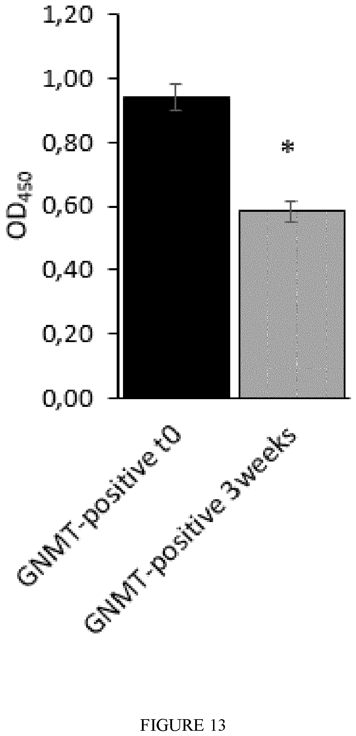

**Figure 13****.** GNMT urine levels determined by ELISA showed a significant reduction 3 weeks after stopping this dietary supplement.

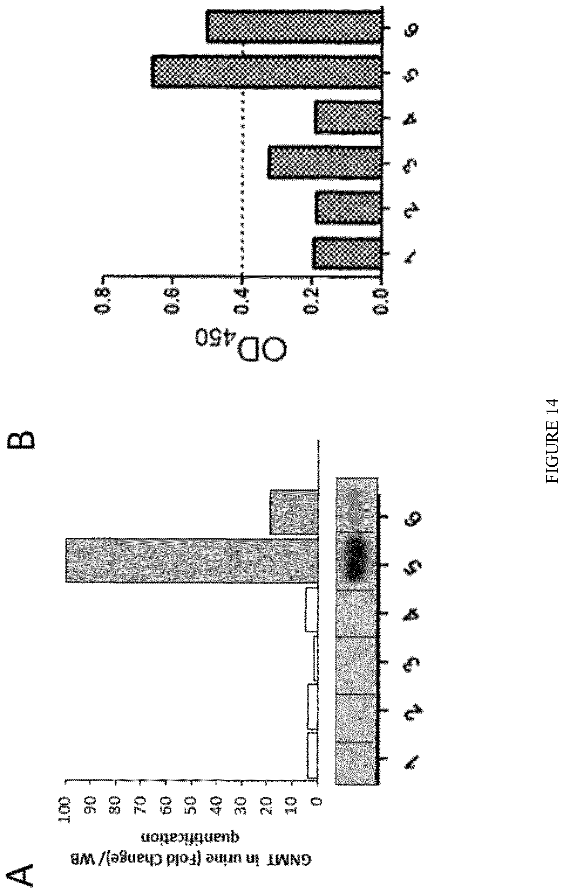

**Figure 14****.** A) GNMT determination by Western Blot in different human urine samples, quantification by densitometry over the chart B) GNMT determination by ELISA method was performed in the same population.

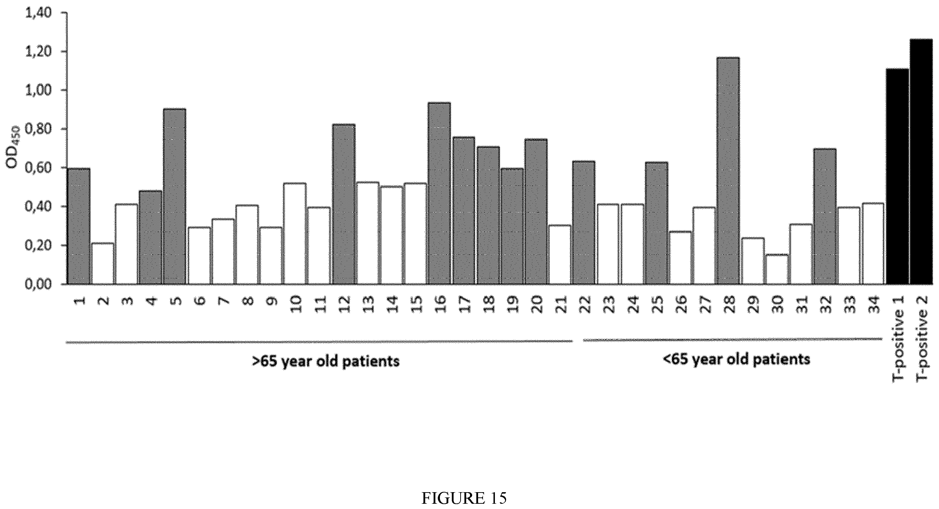

**Figure 15****.** GNMT quantification in urine samples by ELISA in a cohort of different ages. As observed, GNMT positive samples are independent from age.

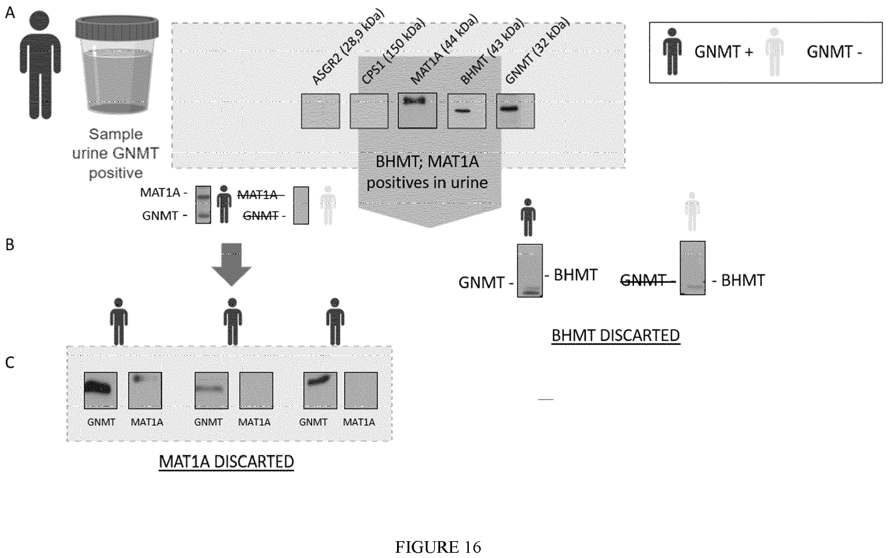

**Figure 16****.** Urine biomarkers screening by WB. A) First urine screening was performed using biological urine fluid from previously known fat-burner GNMT positive patient. B) Positive biomarkers were compared in different negative and positive patients. Nonspecific biomarkers were discarded. C) MAT1A and GNMT comparison in general population. MAT1A is not present at the same way as GNMT, this therefore discarded.

### DETAILED DESCRIPTION OF THE INVENTION

As explained above, the inventors have surprisingly found that elevated urine levels of GNMT can be used as an early onset biomarker for diagnosing several types of liver disease, even before an increase in serum aminotransferase activity due to release of ALT and AST can be detected.

Thus, in a first aspect, the invention refers to an *in vitro* method for diagnosing liver disease in a subject and/or for triaging a subject for liver disease, wherein the method comprises:
a) determining the expression level of GNMT in a urine sample from said subject, and
b) comparing said expression level of GNMT with a reference value,
wherein an increased expression level of GNMT in said urine sample with respect to the reference value is indicative that the subject suffers from liver disease,.

As used herein, a "liver disease" is a liver injury, or an acute or chronic damage to the liver, usually caused by infection, injury, exposure to drugs or toxic compounds, alcohol, impurities in foods, and the abnormal build-up of normal substances in the blood, an autoimmune process, or by a genetic defect (such as haemochromatosis). Sometimes the exact cause of the injury may not be known. Liver disease can be classified as acute or chronic liver disease based in the duration of the disease. In acute liver disease, such as acute hepatitis and acute liver failure (ALF), the history of the disease does not exceed six months. Liver diseases of longer duration are classified as chronic liver disease. Non-limiting examples of common liver diseases include cirrhosis, liver fibrosis, non-alcoholic fatty liver disease (NAFLD), non-alcoholic steatohepatitis (NASH), primary biliary cirrhosis (PBC), and hepatitis, including viral and alcoholic hepatitis. Most common forms of viral hepatitis are hepatitis B and C (HBV and HCV, respectively). Chronic hepatitis may result in cirrhosis. The death of liver cells through a process known as apoptosis is common in all forms of liver disease. Apoptosis of liver cells is linked to liver fibrosis and other liver disease. Accordingly, as used herein, "acute disease" means a condition which is severe and sudden in onset, i.e., it develops over a short period of time. As used herein, "chronic disease" means a condition which develops over a long period of time. Acute diseases may lead to chronic diseases. In the context of the present invention, a liver disease may also include mechanical injuries to the liver, such as hepatic ischemia reperfusion injury.

Liver disease and, in particular, liver disease caused by drugs (drug-induced liver injury or DILI), manifests itself clinically in a variety of symptoms which as such may not be particular informative. Non-limiting examples of symptoms include: loss of appetite, exhaustion, giddiness, weight loss, nausea, vomiting, fever, pain in the upper right abdominal region, arthralgias, myalgias, itching, rashes, discoloration of excretions may be mentioned, jaundice, yellowing of the eyes and of the skin.

As an expert in the field knows, the presence of active liver disease is often detected by the existence of elevated enzyme levels in the blood. Specifically, blood levels of ALT (alanine aminotransferase) and AST (aspartate aminotransferase) above clinically accepted normal ranges are known to be indicative of on-going liver damage. Routine monitoring of liver disease patients for blood levels of ALT and AST is used clinically to measure progress of the liver disease while on medical treatment. Reduction of elevated ALT and AST to within the accepted normal range is taken as clinical evidence reflecting a reduction in the severity of the patient on-going liver damage. In a particular embodiment, the liver disease is caused by any kind of liver damage. However, it must be noted that blood levels of ALT and AST are not always elevated in all types of liver disease, particularly in cases of liver disease that are chronic, but not yet severe.

The term "liver damage", as used herein, is used to denote any type of hepatic trauma (injury), including chronic and acute trauma as well as pathological change present in liver cell or tissue. The clinical conditions of liver damage may include, without being limited thereto, degeneration of live cells, vasculitis of liver, spotty necrosis or focal necrosis present in liver, inflammatory cell infiltration or fibroblast proliferation in liver and portal area, or hepatomegaly, and hepatocirrhosis, hepatoma resulted from severe liver damage, and the like. Liver disease results from an injury to the liver. The injury to the liver may be caused by toxins, including alcohol, some drugs, impurities in foods, the abnormal build-up of normal substances in the blood, by infection or by an autoimmune disorder. The liver damage may also be caused by a mechanical injury to the liver, In some cases, the liver damage resulting from an injury to the liver include, but is not limited to fatty liver, cirrhosis, primary biliary cirrhosis, primary sclerosing cholangitis, and alphal- antitrypsin deficiency. The liver damage includes, but is not limited to cirrhosis, liver fibrosis, non-alcoholic fatty liver disease (NAFLD), non-alcoholic steatohepatitis (NASH), hepatic ischemia reperfusion injury, hepatitis, including viral and alcoholic hepatitis and primary biliary cirrhosis (PBC).

In a particular embodiment, the liver disease is selected from liver injury, acute liver disease, and chronic liver disease. In another particular embodiment, the liver disease includes any of the stages known in liver disease progression: Steatosis, inflammation, fibrosis, cirrhosis and cancer as the end stage. As the person skilled in the art of the invention would readily understand, the damage to a patient's liver is likely to progress in a similar way irrespective of the specific type of liver disease (liverfoundation.org/for-patients/about-the-liver/the-progression-of-liver-disease/). Depending on the specific liver disease in a subject, said subject may suffer from a different disease stage (i.e., cirrhosis may be compensated (stages 1 and 2) or decompensated (stages 3 and 4); Vanesa Bernal, Jaume Bosch, cirrosis hepática, Libro de Gastroenterologia y Hepatologia. Problemas comunes en la práctica clínica. 2^{a} Edición 2019).

In a particular embodiment, the liver disease is selected from the group consisting of drug-induced liver injury (DILI), non-alcoholic fatty liver disease (NAFLD), non-alcoholic steatohepatitis (NASH), liver fibrosis, steatosis, hepatitis, cirrhosis, hepatocellular carcinoma (HCC), veno-occlusive liver disease, Budd-Chiari syndrome, or any combination thereof. In a more particular embodiment, the liver disease is selected from the group consisting of drug-induced liver injury (DILI), non-alcoholic fatty liver disease (NAFLD), non-alcoholic steatohepatitis (NASH), liver fibrosis, steatosis and cirrhosis.

As used herein "non-alcoholic fatty liver disease" (NAFLD) is a condition characterised by an abnormal accumulation of fat in the liver, wherein the abnormal fat accumulation is not caused by excessive alcohol use. There are two types: non-alcoholic fatty liver (NAFL) and non-alcoholic steatohepatitis (NASH). Non-alcoholic fatty liver usually does not progress to liver damage or NASH. NASH includes both a fatty liver and liver inflammation. It may lead to complications such as cirrhosis, liver cancer, liver failure, or cardiovascular disease.

As used herein, "fibrosis" is the formation of excess fibrous connective tissue in an organ or tissue in a reparative or reactive process. This can be a reactive, benign, or pathological state. In response to injury, this is called scarring, and if fibrosis arises from a single cell line, this is called a fibroma. Physiologically, fibrosis acts to deposit connective tissue, which can interfere with or totally inhibit the normal architecture and function of the underlying organ or tissue. Fibrosis can be used to describe the pathological state of excess deposition of fibrous tissue, as well as the process of connective tissue deposition in healing. Defined by the pathological accumulation of extracellular matrix (ECM) proteins, fibrosis results in scarring and thickening of the affected tissue, it is in essence an exaggerated wound healing response which interferes with normal organ function. Fibrosis may affect any organ. In a preferred embodiment, fibrosis is liver fibrosis. As used herein "liver fibrosis" is the scarring process that represents the liver's response to injury. In the same way as skin and other organs heal wounds through deposition of collagen and other matrix constituents so the liver repairs injury through the deposition of new collagen. Liver fibrosis is the first stage of liver scarring. Later, if more of the liver becomes scarred, it may lead to liver cirrhosis.

As used herein "veno-occlusive liver disease" or "hepatic sinusoidal obstruction syndrome (SOS)" is characterized by hepatomegaly, right upper quadrant pain, jaundice, and ascites, most often occurring in patients undergoing hematopoietic cell transplantation (HCT) and less commonly following the use of certain chemotherapeutic agents in non-transplant settings, ingestion of alkaloid toxins, after high dose radiation therapy, or liver transplantation. The hepatic venous outflow obstruction in SOS is due to occlusion of the terminal hepatic venules and hepatic sinusoids

As used herein "drug-induced liver injury (DILI)" means injury to the liver which develops following the use of one or more drugs. Adverse drug reactions are an important cause of liver injury that may require discontinuation of the offending agent, hospitalization, or even liver transplantation. Because the liver is responsible for concentrating and metabolizing a majority of medications, it is a prime target for medication-induced damage. Among hepatotoxic drugs, acetaminophen (paracetamol) is the most often studied. However, a broad range of different pharmacological agents can induce liver damage, including anesthetics, anticancer drugs, antibiotics, antituberculosis agents, antiretrovirals, and cardiac medications. Depending on the duration of injury and the histological location of damage, drug-induced liver injury (DILI) is categorized as acute or chronic, and either as hepatitis, cholestatic, or a mixed pattern of injury. In the context of the present invention, a drug-induced liver injury may include herb-induced liver injury (HILI). It should be noted that drug consumption in small quantities may induce chronic mild disease. This type of chronic mild liver injury is not considered a drug induced liver injury.

As used herein "steatosis", also called fatty change, is the process describing the abnormal retention of lipids within a cell. It reflects an impairment of the normal processes of synthesis and elimination of triglyceride fat. Excess lipid accumulates in vesicles that displace the cytoplasm. When the vesicles are large enough to distort the nucleus, the condition is known as macrovesicular steatosis; otherwise, the condition is known as microvesicular steatosis. While not particularly detrimental to the cell in mild cases, large accumulations can disrupt cell constituents, and in severe cases the cell may even burst. The risk factors associated with steatosis are varied, and include diabetes mellitus, protein malnutrition, hypertension, cell toxins, obesity, anoxia, and sleep apnea.[ As the liver is the primary organ of lipid metabolism it is most often associated with steatosis; however, it may occur in any organ, commonly the kidneys, heart, and muscle. In a preferred embodiment, the steatosis is caused in the liver.

As used herein "non-alcoholic steatohepatitis (NASH)" is a syndrome that develops in patients who are not alcoholic; it causes liver damage that is histologically indistinguishable from alcoholic hepatitis. It develops most often in patients with at least one of the following risk factors: obesity, dyslipidemia, and glucose intolerance. Pathogenesis is poorly understood but seems to be linked to insulin resistance (i.e., as in obesity or metabolic syndrome). Most patients are asymptomatic. Laboratory findings include elevations in aminotransferase levels. Biopsy is required to confirm the diagnosis.

As used herein "cirrhosis", means a late stage of progressive hepatic fibrosis characterized by distortion of the hepatic architecture and the formation of regenerative nodules. It is generally considered to be irreversible in its advanced stages, at which point the only treatment option may be liver transplantation. Reversal of cirrhosis (in its earlier stages) is possible in several forms of liver disease following treatment of the underlying cause. Patients with cirrhosis are susceptible to a variety of complications, and their life expectancy is usually markedly reduced.

As used herein "hepatocellular carcinoma (HCC)", means an aggressive tumour that often occurs in the setting of chronic liver disease and cirrhosis. It is typically diagnosed late in its course, and the median survival following diagnosis is approximately 6 to 20 months. Although the mainstay of therapy is surgical resection, the majority of patients are not eligible because of tumour extent or underlying liver dysfunction.

As used herein "Budd-Chiari syndrome" (BCS) is defined as hepatic venous outflow tract obstruction, independent of the level or mechanism of obstruction, provided the obstruction is not due to cardiac disease, pericardial disease, or sinusoidal obstruction syndrome (veno-occlusive disease). Primary Budd-Chiari syndrome is present when there is obstruction due to a predominantly venous process (thrombosis or phlebitis), whereas secondary Budd-Chiari is present when there is compression or invasion of the hepatic veins and/or the inferior vena cava by a lesion that originates outside of the vein (eg, a malignancy).

As used herein "hepatitis" means an inflammation of the liver, irrespective of the cause. Hepatitis may be caused by a number of conditions, including drug toxicity, immune diseases, and viruses. It is characterized by jaundice, liver enlargement, and fever.

In a preferred embodiment, the expression levels of alanine aminotransferase (ALT) and/or aspartate aminotransferase (AST) in the urine sample from the subject are not increased with respect to a reference value (i.e., the subject has normal levels of AST and/or ALT in serum). Accordingly, detection of GNMT levels can be used as a marker of early stages of liver damage.

The term "diagnosis" as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. Accordingly, the term "diagnosis" as used herein includes the medical triage of patients. Triage is the process of determining the priority of patients' treatments by the severity of their condition or likelihood of recovery with and without treatment. As the person skilled in the art will understand, such a diagnosis may not be correct for 100% of the subject to diagnose, although preferably it is. The term, however, requires that a statistically significant part of the subjects can be identified as suffering from a disease, particularly a liver disease, a kidney disease or a lung disease in the context of the invention, or having a predisposition thereto. The skilled in the art may determine whether a party is statistically significant using different statistical evaluation tools well known, for example, by determination of confidence intervals, the p-value determination, Student's-test, the Mann- Whitney, etc. (see Dowdy and Wearden, 1983). Preferred confidence intervals are at least, 50%, at least 60%, at least 70%>, at least 80%>, at least 90%) or at least 95%. The p-values are preferably, 0.05, 0.025, 0.001 or lower. The term "diagnosing" as disclosed herein means the process of determining which disease or condition explains a person's symptoms and signs.

The terms "subject", "patient" or "individual" are used herein interchangeably to refer to any member of the animal kingdom and can be a vertebrate, such as, a fish, a bird, a reptile, an amphibian or a mammal, including a human, non-human primate, horse, pig, rabbit, dog, sheep, goat, cow, cat, guinea pig or rodent. Preferably, the subject is a mammal, more preferably a rat, a mouse or a human. In a more preferred embodiment, the subject is a human.

As it is used herein, the term "GNMT" means glycine N-methyltransferase. In the human genome, it corresponds to the human gene identified by ID number ENSG00000124713 in the Ensembl database. In the mouse genome, it corresponds to the mouse gene identified by ID number ENSMUSG00000002769 in the Ensembl database.

As used herein, "urine sample" refers to a liquid sample of undiluted or dilutes urine.

Methods suitable for determining the expression level of GNMT are known in the art, and include any method suitable for determining the expression of the GNMT protein levels or for determining the activity level of the GNMT protein.

Methods suitable for determining the expression level of GNMT protein include, without limitation, quantification by means of conventional methods, for example, using antibodies capable of binding specifically to GNMT protein and the subsequent quantification of the resulting antibody-antigen complexes. There is a wide range of well-known assays which can be used in the present invention, using non-labelled antibodies (primary antibody) and labelled antibodies (secondary antibody); these techniques include, among others, Western blot, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of biochips or protein microarrays including specific antibodies, or assays based on colloidal precipitation in formats such as dipsticks. Other ways of detecting and quantifying the levels of the protein of interest include affinity chromatography techniques, ligand binding assays, etc. Non-limiting examples of immunoassays include lateral flow assays. In a particular embodiment, the GNMT expression level is determined by a method selected from the group consisting of Enzyme Linked Immunosorbent Assay (ELISA), Lateral Flow Assay (LFA), Point of Care Instrument (PoC), Western blot, immunoassay, microarray, mass spectrometry, surface plasmon resonance (SPR), Magnetic Resonance, fluorescent assays and Förster Resonance Energy Transfer (FRET). In a preferred embodiment the expression level of GNMT protein is determined by an immunoassay. In a more preferred embodiment the expression level of GNMT protein is determined by a lateral flow immunoassay.

In the context of the present invention, a lateral flow assay (LFA) is a paper-based platform for the detection and quantification of analytes in complex mixtures, where the sample is placed on a test device and the results are displayed on average within 5-30 min. LFA-based tests may be used both for the qualitative and quantitative detection of specific antigens and antibodies, as well as products of gene amplification. A variety of biological samples can be tested using LFAs, including urine. Depending on the elements of recognition used, various lateral flow assay methods may be utilized to test for the presence or absence or quantity of an analyte in a biological sample. In one example, a "sandwich" assay method uses an antibody immobilized on a solid support, which forms part of a complex with a labelled antibody, to determine the presence of a target analyte by observing the presence and amount of bound analyte-labelled antibody complex. Lateral flow devices may be available in numerous different configurations. In a non-limiting example, a test strip may include a flow path from an upstream sample application area to a test site, such as from a sample application area through a mobilization zone to a capture zone. The mobilisation zone may contain a mobilisable marker that may interact with the protein of interest, and the capture zone may contain a reagent that binds the protein of interest for detection and/or quantification. In other embodiments, exemplary sample collection kits may include an absorbent medium, such as filter paper, that may include indicia for the placement of the test sample on the medium. Such kits also may include a mailer for sending the test sample to a physician or laboratory for analysis.

Conventional lateral flow test strips feature a solid support on which the sample receiving area and the target capture zones are supported. The solid support material is one which is capable of supporting the sample receiving area and target capture zones and providing for the capillary flow of sample out from the sample receiving area to the target capture zones when the lateral flow test strip is exposed to an appropriate solvent or buffer which acts as a carrier liquid for the sample. General classes of materials that may be used as support include organic or inorganic polymers, and natural and synthetic polymers. More specific examples of suitable solid supports include, without limitation, glass fibre, cellulose, nylon, crosslinked dextran, various chromatographic papers and nitrocellulose. When antibodies are exclusively used as recognition elements the LFA is a 'lateral flow immunoassays' (LFIAs). According to the invention the LFIA may be used in a lateral flow device. For the purposes of a lateral flow immunoassay, the label may be an enzyme, coloured microspheres, fluorescently-labelled microspheres, or may use other similar detection methods that provide for detection and/or quantification of analyte bound to the test line.

"Reference value", as used herein relates to a laboratory value used as a reference for the values/data obtained from samples. The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample, such as, for example, a value obtained from a sample of study but obtained at a previous point in time. The reference value can be based on a high number of samples, such as the values obtained in a population of samples or based on a pool of samples including or excluding the sample to be tested. In a particular embodiment, the reference value for the GNMT level is the GNMT level in healthy subjects who do not suffer from a disease or a particular phenotype. For example, the reference value can be based on the expression level of the marker to be analysed obtained from subjects who do not need treatment for a liver disease, preferably from healthy subjects who are not suffering from any disease.

The expression of a protein is considered to be increased when its levels increase with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% or more. For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. The reference value refers to the level of protein in a control subject or subjects, which may be a subject or subjects who do not suffer from a specific disease and who are generally considered to be healthy. In the context of the present invention, the reference value refers to the protein level of GNMT in a control subject. In an embodiment, the control subject is a subject who does not suffer from a liver disease. In an embodiment, the control subject is a subject who does not have any symptoms of liver disease. In a particular embodiment, the protein level of GNMT in the control subject population is undetectable (i.e., absent) because it is under detection levels in a urine sample. Thus, merely detecting the presence of GNMT in the urine is sufficient for a finding that the subject suffers from a liver disease. Accordingly, the methods of the invention allow for the detection of the presence or the absence of the GNMT protein in the urine, and to associate this result with a finding that the subject suffers from a liver disease if the GNMT protein is present in the urine, or with a finding that the subject does not suffer from a liver disease if the GNMT protein is absent from the urine.

As the skilled person surely notices, determining the activity level of the GNMT protein would offer an alternative, indirect method to assess the expression level of the GNMT protein. Thus, in a particular embodiment, the activity level of the GNMT protein is determined, that is, the activity levels of the protein encoded by the GNMT gene are determined. The term "activity level" of a protein, more particularly of an enzyme, as used herein refers to a measure of the enzyme activity, particularly measured as moles of substrate converted per unit of time. Assays to determine the activity level of an enzyme are known by the skilled person and include, without limitation, initial rate assays, progress curve assays, transient kinetics assays and relaxation assays. Continuous assays of enzymatic activity include, without limitation, radiometric, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration. A non-limiting example of an assay to determine the activity level of the GNMT protein is described in DebRoy S, Kramarenko II, Ghose S, Oleinik NV, Krupenko SA, Krupenko NI (2013) A Novel Tumor Suppressor Function of Glycine N-Methyltransferase Is Independent of Its Catalytic Activity but Requires Nuclear Localization. PLoS ONE 8(7): e70062.

In a further aspect, the invention refers to an *in vitro* method for monitoring progression of liver disease in a subject, wherein the method comprises:
a) determining the expression level of GNMT in a urine sample from said subject at a first period of time;
b) determining the expression level of GNMT in a bio urine fluid sample from said subject at a second period of time, wherein said second period of time is later than said first period of time; and
c) comparing the expression level of GNMT at the second period of time with the expression level of GNMT at the first period of time,
wherein an increased expression level of GNMT at the second period of time with respect to the expression level of GNMT at the first period of time is indicative that the liver disease is progressing in the subject; wherein a decreased or an equal expression level of GNMT at the second period of time with respect to the expression level of GNMT at the first period of time is indicative that the liver disease is not progressing in the subject.

In yet a further aspect, the invention relates to an *in vitro* method for monitoring the response to a therapy in a subject suffering from liver disease, wherein the method comprises:
a) determining the expression level of GNMT in a urine sample from said subject prior to the administration of said therapy;
b) determining the expression level of GNMT in a urine sample from said subject during or after the administration of said therapy; and
c) comparing the expression level of GNMT during or after the administration of said therapy with the expression level of GNMT prior to the administration of said therapy,
wherein a decreased expression level of GNMT during or after the administration of said therapy with respect to the expression level of GNMT prior to the administration of said therapy is indicative that the subject has a positive response to the therapy; wherein an increased or an equal expression level of GNMT during or after the administration of said therapy with respect to the expression level of GNMT prior to the administration of said therapy is indicative that the subject does not have a positive response to the therapy.

The expression level of GNMT at the second period of time, or during or after the administration of a therapy, is considered to be increased with respect to the expression level of GNMT at the first period of time, or prior to the administration of said therapy, when said level at the second period of time, or during or after the administration of a therapy, increases with respect to said level at the first period of time, or prior to the administration of said therapy, by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% or more. For example, an increase in expression levels of at least 1.1-fold, 1.5-fold, 5-fold, 10-fold, 20-fold, 30-fold, 40-fold, 50-fold, 60-fold, 70-fold, 80-fold, 90-fold, 100-fold or even more is considered as "increased" expression level.

The term "treatment" or "therapy", as used herein, refers to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

Treatment for liver disease depends on a patient's diagnosis. Some liver problems can be treated with lifestyle modifications, such as stopping alcohol use or losing weight, typically as part of a medical program that includes careful monitoring of liver function (i.e. preventive treatments are susceptible to being monitored). Other liver problems may be treated with medications or may require surgery. Accordingly, treatment of liver disease may include administration of a suitable type of pharmacological substance, or it may include lifestyle changes in a patient's diet, exercise routine, alcohol intake, etc. Pharmacological treatments typically need to be carefully monitored for putative adverse events associated with any administered treatment. If adverse effects associated with a pharmacological treatment are detected, treatment is discontinued. Treatments include, but are not limited to, dexamethasone, pentasa, mesalazine, asacol, codeine phosphate, benorylate, fenbufen, naprosyn, diclofenac, etodolac, indomethacin, aspirin, ibuprofen, as well as non-steroidal antiinflammatory agents (NSAIDS).

Treatments for any kind of liver disease known in the state of the art include, but are not limited to, cholic acid, cholbam, neomycin, paromomycin, humatin, neo-tab, neo-fradin, nexavar, sorafenib, phenobarbital, luminal, ursodiol, actigall, urso, colchicine, urso forte, azathioprime, obeticholic acid, ocaliva, peppermint, floxuridine, FUDR, actos, pioglitazone, actigall, avandia, orlistat, betaine, cystadane, rosiglitazone, creon, pancrelipase, zenpep, pancreaze, viokase, viokace, pertzye, propranolol, nadolol. Chemotherapeutic agents include, but are not limited to, radioactive molecules, toxins, also referred to as cytotoxins or cytotoxic agents, which includes any agent that is detrimental to the viability of cells, agents, and liposomes or other vesicles containing chemotherapeutic compounds (1-dehydrotestosterone, 5-fluorouracil decarbazine, 6-mercaptopurine, 6-thioguanine, actinomycin D, adriamycin, aldesleukin, alkylating agents, allopurinol sodium, altretamine, amifostine, anastrozole, anthramycin (AMC)), anti-mitotic agents, cis-dichlorodiamine platinum (II) (DDP) cisplatin), diamino dichloro platinum, anthracyclines, antibiotics, antimetabolites, asparaginase, BCG live (intravesical), betamethasone sodium phosphate and betamethasone acetate, bicalutamide, bleomycin sulfate, busulfan, calcium leucovorin, calicheamicin, capecitabine, carboplatin, lomustine (CCNU), carmustine (BSNU), Chlorambucil, Cisplatin, Cladribine, Colchicin, conjugated estrogens, Cyclophosphamide, Cyclothosphamide, Cytarabine, Cytarabine, cytochalasin B, Cytoxan, Dacarbazine, Dactinomycin, dactinomycin (formerly actinomycin), daunirubicin HCL, daunorucbicin citrate, denileukin diftitox, Dexrazoxane, Dibromomannitol, dihydroxy anthracin dione, Docetaxel, dolasetron mesylate, doxorubicin HCL, dronabinol, *E. coli* L-asparaginase, emetine, epoetin-.alpha., Erwinia L-asparaginase, esterified estrogens, estradiol, estramustine phosphate sodium, ethidium bromide, ethinyl estradiol, etidronate, etoposide citrovorum factor, etoposide phosphate, filgrastim, floxuridine, fluconazole, fludarabine phosphate, fluorouracil, flutamide, folinic acid, gemcitabine HCL, glucocorticoids, goserelin acetate, gramicidin D, granisetron HCL, hydroxyurea, idarubicin HCL, ifosfamide, interferon alpha. -2b, irinotecan HCL, letrozole, leucovorin calcium, leuprolide acetate, levamisole HCL, lidocaine, lomustine, maytansinoid, mechlorethamine HCL, medroxyprogesterone acetate, megestrol acetate, melphalan HCL, mercaptopurine, mesna, methotrexate, methyltestosterone, mithramycin, mitomycin C, mitotane, mitoxantrone, nilutamide, octreotide acetate, ondansetron HCL, paclitaxel, pamidronate disodium, pentostatin, pilocarpine HCL, plimycin, polifeprosan 20 with carmustine implant, porfimer sodium, procaine, procarbazine HCL, propranolol, rituximab, sargramostim, streptozotocin, tamoxifen, taxol, teniposide, tenoposide, testolactone, tetracaine, thioepa chlorambucil, thioguanine, thiotepa, topotecan HCL, toremifene citrate, trastuzumab, tretinoin, valrubicin, vinblastine sulfate, vincristine sulfate, and vinorelbine tartrate. TNF-a agonist and TNF-a receptors (like etanercept; ENBREL^{®}; Immunex or infliximab called REMICADE^{®}; Centacor) vaccine CytoTAb (Protherics); antisense molecule 104838 (ISIS); the peptide RDP-58 (SangStat); thalidomide (Celgene); CDC-801 (Celgene); DPC-333 (Dupont); VX-745 (Vertex); AGIX-4207 (AtheroGenics); ITF-2357 (Italfarmaco); NPI-13021 -31 (Nereus); SCIO- 469 (Scios); TACE targeter (Immunix/AHP); CLX-120500 (Calyx); Thiazolopyrim (Dynavax); auranofin (Ridaura) (SmithKline Beecham Pharmaceuticals); quinacrine (mepacrine dichlorohydrate); tenidap (Enablex); Melanin (Large Scale Biological); and anti-p38 MAPK agents.

Therapeutic agents made from fragments of lactic bacteria which are intended to stimulate the immune system are also contemplated in the context of the invention. Macrocytic antibiotics (i.e., rapamycin and its analogues, derivatives and cogeners) are also considered, as well as other immunophilins that possesses the same pharmacologic properties, or immunosuppresives.

In addition, therapeutic agents are considered alone or in combination with at least another different agent such as angiogenic agents, fibroblast growth factors, angiotensin receptor blockers (such as nitric oxide, anti-sense oligonucleotides and other combinations) cell cycle inhibitors, mTOR inhibitors, and growth factor receptor signal transduction kinase inhibitors: retenoids; cyciin/CDK inhibitors; HMG co-enzyme reductase inhibitors (statins); and protease inhibitors.

The term "progression", as used herein, is defined as an increase in the severity of symptoms associated with liver disease. Any other parameter which is widely accepted for comparing the efficacy of alternative treatments can be used for determining a response to treatment and include, without limitation:
- Disease-free progression which, as used herein, describes the proportion of subjects in complete remission from liver disease who have had no recurrence of disease during the time period under study.
- Disease-free survival (DFS), as used herewith, is understood as the length of time after treatment for a disease during which a subject survives with no clinical sign of the disease.
- Objective response which, as used in the present invention, describes the proportion of treated subjects in whom a complete or partial response is observed.
- Liver disease control, which relates to the proportion of treated subjects in whom complete response, partial response, minor response or stable disease > 6 months is observed.
- Progression free survival which, as used herein, is defined as the time from start of treatment to the first clinical signals of recurrence of liver disease.
- Time to progression (TTP), as used herein, relates to the time after a disease is treated until the disease starts to get worse.
- Six-month progression free survival or "PFS6" rate which, as used herein, relates to the percentage of subjects wherein free of progression in the first six months after the initiation of the therapy and
- Median survival which, as used herein, relates to the time at which half of the subjects enrolled in the study are still alive.

In a particular embodiment of the first method of the invention, the clinical response is measured as time to progression/ regression/ partial regression or a progression-free survival.

The expression "positive response" when referred to the treatment for a liver disease, as used herein, refers to any response which is substantially better than that obtained with a saline control or placebo. Positive clinical response may also be expressed in terms of various measures of clinical outcome as defined elsewhere herein. Positive clinical outcome can also be considered in the context of an individual's outcome relative to an outcome of a population of patients having a comparable clinical diagnosis, and can be assessed using various endpoints.

In an additional aspect, the invention refers to a method for selecting a subject to be treated for liver disease, wherein the method comprises:
a) determining the expression level of GNMT in a urine sample from said subject, and
b) comparing said expression level of GNMT with a reference value,
wherein an increased expression level of GNMT in said urine sample with respect to the reference value is indicative that the subject is selected for treatment of liver disease.

In a preferred embodiment, this aspect of the invention further comprises the determination of one or more clinical parameters which are also indicative of the prognosis of liver disease. Such indicators include the presence or levels of other known markers for liver disease.

In another aspect, the invention relates to a method of treatment of liver disease in a patient in need thereof, wherein the method comprises the administration of a therapy to said patient, wherein the patient has been selected using a method which comprises the determination of the expression level of GNMT in a urine sample from the patient, wherein an increased expression level of GNMT in said urine sample with respect to the reference value is indicative that the subject is selected for treatment of liver disease.

In another aspect, the invention relates to a method for diagnosing and treating a subject who suffers from a liver disease, the method comprising:
a) obtaining a urine sample from said subject;
b) determining the expression level of GNMT in said urine sample from said subject;
c) comparing the expression level of GNMT with a reference value; wherein an increased expression level of GNMT in said urine sample with respect to the reference value is indicative that the subject suffers from liver disease; and
d) administering a treatment to the subject if the subject is diagnosed as suffering from a liver disease.

In another additional aspect, the invention refers to the use of reagents specific for determining the expression level of GNMT in a urine sample from a subject, for *in vitro* diagnosing a liver disease in said subject.

The reagents specific for determining the expression level of GNMT may be presented in a kit format. In the context of the present invention, "kit" or "assay device" is understood as a product or device containing the different reagents necessary for carrying out the method of determining the expression level of GNMT, packed so as to allow their transport and storage. Materials suitable for packing the components of the kit include crystal, plastic (polyethylene, polypropylene, polycarbonate and the like), bottles, vials, paper, envelopes and the like. Additionally, the kits can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions. In a preferred embodiment, the reagents adequate for the determination of the expression levels of GNMT comprise at least 10%, at least 20%, at least 30%, at least 40%, at least 50%, at least 60%, at least 70%, at least 80%, at least 90% or at least 100% of the total amount of reagents adequate for the determination of the expression levels of genes forming the kit.

The expression "reagent specific for determining the expression level of GNMT" means a compound or set of compounds that allows determining the expression level of GNMT by means of the determination of the level of the GNMT protein. Thus, include compounds that bind specifically with the proteins encoded by the marker genes and preferably include antibodies, although they can be specific aptamers. In the alternative, the expression "reagent specific for determining the expression level of GNMT" means a compound or set of compounds that allows determining the expression level of GNMT by means of the determination of the activity of the GNMT protein. Compounds related to one carbon metabolism or to the methionine cycle may be used as suitable reagents for determining the expression level of GNMT. In a particular embodiment, the reagents specific for determining the expression level of GNMT comprise sarcosine and/or S-adenosylmethionine(SAM) (AdoMet). In the context of the invention, the reagents specific for determining the activity level of GNMT may include activity buffers. One suitable activity buffer is, without limitation, Tris 40mM pH 8 / pH 9 * NaCl * 10mM MgCl2. 50 µL of liver homogenate. 100µL (2mM Gly *labeled /200µM SAMe in buffer) 100 µL water/urine.

All the terms and embodiments described herein are equally applicable to all aspects of the invention.

The invention will also be described by way of the following examples, which are to be considered as merely illustrative and not limitative of the scope of the invention.

### EXAMPLES

### Materials and methods

### Human samples

All the studies were performed in agreement with the Declaration of Helsinki and according to local national laws. The Human Ethics Committee of each hospital approved the study procedures and written informed consent was obtained from all patients before inclusion in the study. Serum and urine samples were obtained from two different cohorts of patients diagnosed in Marqués de Valdecilla University Hospital (MVUH, Santander, Spain). For glycine N-methyltransferase (GNMT) determination by Western Blot in Figure A, there were used serum samples from a cohort of 8 healthy people and 45 patients diagnosed through biopsy with different grades of liver disease (steatosis, inflammation, ballooning and fibrosis). For GNMT determination by Western Blot in Figure C, there were used urine samples from a cohort of 17 healthy people and 13 patients with liver disease previously diagnosed or determined by the grade of fibrosis through Fibroscan. For GNMT determination by enzyme-linked immunosorbent assay in Figure D, there were used urine samples from a cohort of 390 samples either with no alterations or different grades of liver injury measured through Fibroscan.

### Animal experiments

All the animal experiments were conducted in accordance with the Spanish Guide for Care and use of Laboratory animals, and with the International Care and Use Committee Standards. All procedures were approved by the CIC bioGUNE's Animal Care and Use Committee and the competent authority (Diputación de Bizkaia). Mice were housed in a temperature-controlled animal facility (AAALAC-accredited) within 12-hour light/dark cycles. G-They were fed a standard diet (Harlan Tekland) with water ad libitum.

**DILI animal model:** Adult C57BU6J wilt-type mice were treated with 360mg/kg of acetaminophen (APAP) to induce acute liver injury. After 48h of treatment, mice were sacrificed and liver were split into several pieces for subsequent analysis including: RNA or protein extraction, formalin fixation for histology and immunohistochemistry or metabolic analysis. Blood for serum analysis was collected once a week during the treatment.

**NASH animal model:** C57BL/6J wild-type mice were fed with a methionine (0.1%) and choline (0%) deficient diet for 4 weeks. In case of treated mice, 2 weeks after the beginning of the diet mice were divided in two groups and subjected to A) an in vivo targeting of miR-873-5p (60µg/mouse) (Dharmacon, USA) or an unrelated miR-Ctrl using Invivofectamine ^{®} 3.0 Reagent (Invitrogen, USA) by tail vein injection (Fernandez-Tussy P, Fernández-Ramos D, [...], Martinez-Chantar ML. *miR-873-5p targets mitochondrial GNMT-Complex II interface contributing to non-alcoholic fatty liver disease.* (2019) Molecular Metabolism), or B) once-a-week intragastric administration of 60mg/kg MLN4924 (Millenium Pharmaceuticals, USA). Treatments were performed until the fourth week. At the end of the treatments, animals were sacrificed and liver were split into several pieces for subsequent analysis including: RNA or protein extraction, formalin fixation for histology and immunohistochemistry or metabolic analysis. Blood for serum analysis was collected once a week during the treatment.

**Fibrosis and cirrhosis animal models:** Mice lacking ATP-binding cassette B4 (Mdr2-/-) were used as they develop spontaneously fibrosis and cirrhosis under 3 and 5 months of maintenance respectively (Popov Y, Patsenker E, Fickert P, Trauner M, Schuppan D. Mdr2 (Abcb4)-/- mice spontaneously develop severe biliary fibrosis via massive dysregulation of pro- and antifibrogenic genes. (2005) Journal of Hepatology)

**Starvation animal model:** Adult C57BL/6J mice were maintained in cages feeding them with a standard diet. Mice were starved during 16 and 24 hours.

**Renal fibrosis animal models:** Adult C57BU6J mice were induced renal fibrosis through an acute dose of folic acid (FA) or by unilateral ureteral obstruction (UUO). In FA model folic acid was administered intravenously at a 250mg/kg dose, and mice were sacrificed 14 days after intervention. In case of UUO animal model, one of the ureteral conducts was ligated and mice were sacrificed 5 days after intervention.

### In Vitro Experiments

Primary hepatocytes from 3-month old wild type (C57BL/6J) were isolated by perfusion with collagenase Type I (Worthington, USA). Briefly, animals were anesthetized with isoflurane (1.5% isoflurane in O2). Then, the abdomen was opened and a catheter was inserted into the inferior vena cava. Liver was perfused with buffer A (1x PBS, 5mM EGTA, 37°C and oxygenated) and the portal vein was cut. Next, liver was perfused with buffer B (1x PBS, 1mM CaCl2 37°C and oxygenated) to remove EGTA, and finally perfused with buffer C (1x PBS, 2mM CaCl2, 0.65 BSA, collagenase type I, 37°C and oxygenated). After buffer C perfusion, liver was separated from the resto of the body and placed into a petri dish with MEM (Gibco, USA). Gall bladder was carefully removed and, then, liver was mechanically disaggregated with forceps. The digested liver diluted in MEM was filtered through a sterile gauze and filtered liver cells were collected and washed three times (1x4' at 400RPM and 2x5' at 500RPM) in 10% FBS (Gibco)/1% PSG (Gibco) supplemented MEM, conserving all supernadant Kupffer and Hepatic Stellate cells isolation. After the final wash, hepatocytes contained in the pellet were resuspended in 10% FBS 1% PSG MEM for subsequently culturing. Primary hepatocytes were seeded over previously collagen-coated culture dishes at a density of 7600 cells/mm2 in 10% FBS/1% PSG supplemented MEM and placed in an incubator at 37°C, 5%CO2-95% air. After 6 hours of attachment, culture medium and unattached hepatocytes were removed with fresh 0% FBS/1% PSG MEM. After overnight with media deprived of sera, damaged experimental procedures start with their respective treatments. Conditions indicated in the following table:

| **Reagent** | **Dose** | **Vehicle** | **Time** | **Function** | **%FBS** |
|---|---|---|---|---|---|
| Acetaminophen (APAP) | 10 mM | PBS | 6h | Increase necrosis in hepatocytes | 0% |
| Medium Deficient in Methionine and Choline (MCD) | | | 24 h | Increase lipid content and ROS production in hepatocytes | 0% |
| Oleic acid (OA) | 400 µM | Ethanol | 24h | Increase lipid content in hepatocytes | 0% |

### Western Blotting

Protein extracts were boiled at 95 °C for 5 min in SDS-PAGE sample buffer (250 mM Tris-HCl pH 6.8, 500 mM β-mercaptoethanol, 50% glycerol, 10% SDS and bromophenol blue). An appropriate amount of protein (between 5 and 50 µg), depending on protein abundance and antibody sensitivity, were separated by sodium dodecyl sulphate-polyacrylamide gel electrophoresis (SDS-PAGE) in 3% to 15% acrylamide gels (depending on the molecular weight of the protein of interest), using a Mini-PROTEAN Electrophoresis System (Bio-Rad). Gels were transferred onto nitrocellulose membranes by electroblotting using a Mini Trans-Blot cell (Bio-Rad). Membranes were blocked with 5% nonfat milk in TBS pH 8 containing TBS-T (0.1% Tween-20, Sigma Aldrich) (TBST-0.1%), for 1 hour at RT and incubated overnight at 4 °C with a 1 :1000 dilution of an anti-GNMT primary antibody (produced at home) or anti-ALT primary antibody (Abcam) in 5% milk TBS-T. Membranes were then washed three times during 10' with TBST-0.1% and incubated for 1 hour at RT in 5% milk TBS-T containing a 1:5000 dilution of secondary anti-rabbit antibody conjugated to horseradish-peroxidase (Cell Sinalling). Immunoreactive proteins were detected by using Western Lightning Enhanced Chemiluminescence reagent (ECL, PerkinElmer, USA) and exposed to Super Rx-N X-ray films (Fuji, Japan) in a Curix 60 Developer (AGFA, Belgium).

### Enzyme-linked immunosorbent assay

GNMT was determined by enzyme-linked immunosorbent assay (ELISA). A precoating was realized with 40µl sample and 60 µl coating buffer (Na2CO3 anhydrous, pH 9.6) per well at 4 °C overnight. Well were washed twice with 100µl/well of washing buffer (PBS tween 0.05%) and wells were blocked with blocking buffer (fetal bovine serum 1% in PBS) for 2h. Wells were incubated with the a 1:1000 dilution of primary antibody (anti-GNMT) in blocking buffer during 1h at room temperature. Wells were then washed four times with 100µl/well of washing buffer before 1h incubation of a 1:1000 dilution of secondary antibody (anti-rabbit with HRP) in blocking buffer. Then, wells were washed 6 times with 100µl/well of washing buffer and 2 additional times with 400µl/well of PBS. There were added 100µl per well of substrate solution (1-Sep Turbo TMB-ELISA) and, once the color is appreciable, the reaction was stopped by adding 100µl per well of 2M sulfuric acid. Optical density was determined at 450nm wavelength and the value of the blank was subtracted for each sample.

### Example 1: GNMT is altered in different stages of liver disease preclinical models

GNMT expression was first evaluated in liver tissue from different mice models. A 0.1% methionine and choline-deficient diet was used as a NAFLD preclinical model (Steatosis at early stages, 2 weeks) and NASH (late stages, 4 weeks), Mdr2-/- mice were used as different models of fibrosis (at early stage, 3 month) and cirrhosis (late stages, 5 month), acetaminophen induced liver injury was used to provoke acute liver damage by DILI. The preclinical model Mdr2 (-/-) is a model used for the study of cholestasis in the context of chronic inflammation as a result of increased biliary acids accumulation. Acetaminophen, which is the principal cause of acute liver failure (ALF) in the US and Europe, produce hepatic oxidative stress and mitochondrial dysfunction through NAPQI metabolite. 0.1% methionine and choline-deficient diet or MCD diet is characterized by the absence or deficiency of methionine and choline, two essential amino acids. This deficiency compromises rapid induction of steatosis and inflammation. The method used to determine GNMT expression was Western Blotting using a primary polyclonal antibody specific for the protein. The downregulation of GNMT in the liver correlated with the development of the pathology (Fig. 1). These results are following published work where the downregulation of the protein correlates with liver disease development.

### Example 2: GNMT is present in the serum of preclinical animal models at early stages of liver disease

Nowadays most of the methods for diagnosing steatosis are based on imaging procedures without a liver enzyme and liver function tests validated for this disease. Here, the inventors reach one of the main points that differentiate the present invention from other diagnostic methods: The presence of GNMT in serum. To address this point, the inventors analysed the serum of the same animal models with liver GNMT downregulation. GNMT was identified in serum with the same polyclonal antibody described (Fig. 2), it is also important to highlight that the inventors can detect GNMT at very early stages of liver damage (for instance, steatosis at two weeks of 0.1% methionine and choline-deficient diet, which triggers NAFLD, considered an early stage of liver damage). It must be mentioned that a serum sample of GNMT-/- mice, absent of the protein, was also analyzed in every experiment in order to demonstrate the specificity of the antibody used in serum samples.

In order to exam the sensitivity of GNMT as a marker of early liver damage, the protein was checked in different starvation periods, and serum was analyzed at 16 and 24 hours of starvation. The effect of this intervention is the accumulation of lipids in the liver due to a lipolytic mechanism derived from the adipose tissue with the subsequent liver stress or even by promoting autophagy in hepatocytes in more extended periods. Thus, a mice model of starvation (animals were deprived of food at different periods of time) was studied trying to emulate the mentioned effect at its earliest stages. The presence of GNMT in serum, demonstrate that the inventors can detect harmful liver conditions at earlier stages than NASH. (Fig. 3). Therefore, these data highlighted that levels of GNMT in serum can be studied as a reliable and sensitive early biomarker for preclinical models of liver damage.

### Example 3: GNMT in serum is a liver-specific biomarker

As mentioned before, a suitable biomarker of liver damage must be specific and able to distinguish between the pathologies related and non-related to liver. Regarding this point, GNMT in serum was determined in different animal models such as FA (folic acid nephropathy) or UUO (unilateral urethral obstruction), previously described to develop chronic kidney injury. In the presented figure, it is clear that GNMT can be only detected in serum of liver-specific injuries (Fig. 4). Therefore, the presented results characterize GNMT as a specific serum biomarker for liver pathologies.

### Example 4: GNMT in serum monitors the progression of liver damage

Currently, there is not a suitable routinely test to monitor the progression of liver damage and the effectiveness of therapies. This challenge can be solved with the present invention. Therapeutic approaches in different preclinical models of NAFLD and fibrosis/cirrhosis were performed and reversion of the liver damage was monitored by a reduction of GNMT levels in the serum (Fig. 5). Mice with NASH and fibrosis were treated with two different preclinical methods knowing to have promising results: MLN4924 as inhibitor of NEDD8-Activating Enzyme (NAE) (related with liver fibrosis stages) and RNA therapeutic anti-miR-873-5p that restore GNMT levels in liver and is associated with amelioration in different liver pathological conditions. Here, the inventors use these therapies to treat NASH promoted by 0,1% methionine and choline-deficient diet. Thus, GNMT appears as a robust marker for routinely follow up of treatment response of liver damage.

### Example 5: GNMT can be also detected in in vitro approaches

Drug toxicity is one of the main limiting steps in the development of a new molecule. An enormous effort has been made to find toxicity models that allow rapid discrimination in high throughput screening platform. Therefore, the identification of a suitable biomarker for in vitro approaches will be a remarkable milestone for drug toxicity. Thus, primary mouse hepatocytes were treated with acetaminophen (APAP) overdose as an *in vitro* approach of liver injury, then, culture media was analysed. The response was compared with a non-transformed hepatocyte cell line MLP29, this cell line is known to have no CYPs that metabolise Acetaminophen. All cells were seeded in wells pretreated with collagen type I and were incubated in fresh media with 10% foetal bovine serum (FBS; Gibco). Minimum essential medium (MEM; Gibco) contains penicillin (100 U/ml), streptomycin (100 U/ml), and glutamine (2 mM) (PSG; Invitrogen) during 6 hours until cell adhesion. After that, cells were incubated overnight with MEM at 0% FBS, next day, cells were treated during 1 hour with acetaminophen dissolved in PBS at a dose of 10 mM. After that, supernatants were collected and analysed. The presence of GNMT was higher in primary hepatocytes media under APAP treatment. MLP29 cells, resistant to acute liver damage and dedifferentiation, were analysed as a negative control (Fig. 6). Liver injury was also induced in primary hepatocytes with methionine and choline-deficient medium (MCD) or oleic acid (OA). GNMT and ALT proteins in the culture media of primary hepatocytes were determined by Western Blot analysis following 24h treatment of methionine and choline-deficient medium (MCD) or oleic acid (OA) to induce liver injury due to toxic lipid accumulation. GNMT is detected in the culture medium as a marker for liver injury while ALT does not correlate with the hepatocyte injury (Fig. 7). It is important to mention that primary hepatocytes undergo a rapid dedifferentiation during in vitro processes. In addition, MEM at 0% expose cells to stress. For these reasons, the inventors could explain why GNMT is secreted in cell culture media in hepatocytes without hepatotoxic treatment. Based on these results, GNMT is validated as a suitable biomarker to analyse hepatotoxicity in *in vitro* approaches in the culture medium. This biomarker will help to develop a high throughput toxicity screening for preclinical and clinical studies.

### Example 6: GNMT as a non-invasive biomarker of liver disease

Currently there is a need for routinely non-invasive methods to detect and follow up liver damage. The only available procedures until date are invasive and frequently need a professional to perform those procedures, so developing a non-invasive alternative is needed. Following this premise, urine of different animal models of liver damage was evaluated. Importantly, GNMT was detected in mice subjected to an acute liver injury (partial hepatectomy, PH) or remarkable at early stage of liver disease (NAFLD) when compared to control or GNMT-deficient mice. (Fig. 8). Importantly, the analysis of GNMT levels in urine was able of discriminating stage of disease and reversion as shown in figure 8.

### Example 7: GNMT is a biomarker for human liver damage

GNMT as a routine biomarker for liver damage was validated in human serum samples (see table 1 below). First of all, sera from a cohort of patients with NASH and different grades of steatosis (i.e., steatosis grade 1, 2 or 3), diagnosed by liver biopsy were selected to be analysed by GNMT levels (Fig. 9A). As shown in the figure, there is a correlation between steatosis progression and GNMT levels detected in serum (Fig. 9B).

**Table 1. Characteristics of steatotic patients.**

| | | **STEATOSIS** | | |
|---|---|---|---|---|
| | Population | 1 | 2 | 3 |
| N | 31 | | | |
| Male | 18 (58%) | 5 (27%) | 10 (56%) | 3 (17%) |
| Female | 13 (42%) | 1 (9%) | 7 (54%) | 5 (35%) |
| Age (years) | ±55 | | | |
| ALT/GPT standard values 0-41 U/L | 12 (39%) | 4 (33%) | 8 (67%) | 0 |
| ALT/GPT out of standard values | 19 (61%) | 2 (11%) | 9 (47%) | 8 (42%) |
| AST/GOT standard values 0-37 U/L | 15 (48%) | 4 (27%) | 9 (60%) | 2 (13%) |
| AST/GOT out of standard values | 16 (52%) | 2 (13%) | 8 (50%) | 6 (37%) |
| GGT standard values 9-48 U/L | 9 (29%) | 2 (22%) | 5 (56%) | 2 (22%) |
| GGT out of standard values | 17 (71%) | 2 (12%) | 11 (65%) | 5 (29%) |

| | | **FIBROSIS** | | |
|---|---|---|---|---|
| Grade 0 | 2 (6.4%) | 1 (50%) | 0 | 1 (50%) |
| Grade 1 | 3 (10%) | 0 | 2 (67%) | 1 (33%) |
| Grade 2 | 10 (32%) | 2 (20%) | 7 (70%) | 1 (10%) |
| Grade 3 | 13 (42%) | 2 (15%) | 7 (55%) | 4 (30%) |
| Grade 4 | 3 (10%) | 1 (33%) | 1 (33%) | 1 (33%) |

| | | | | |
|---|---|---|---|---|
| ALT alanine aminotransferase, AST aspartate aminotransferase, GGT gamma glutamyl aminotransferase. | | | | |

Further, the inventors compared the different liver markers in body fluids. The presence of glycine N-methyltransferase (GNMT) was determined by Western Blotting in 52 serum samples belong to liver patients with different etiologies and compared to the clinical parameters aspartate aminotransferase (AST) and alanine aminotransferase (ALT). GNMT appears in the serum of 44/44 patients with liver pathology versus 2/8 healthy samples. ALT and AST are utterly absent in the samples belong to liver pathologies conditions (Fig. 10A). In addition, the activity of the aminotransferases aspartate aminotransferase (AST) and alanine aminotransferase (ALT) was measured in urine samples derived from patients with a GNMT-positive liver pathology. Importantly, in the samples belonging to these patients AST and ALT activities were absent (Fig. 10B). In order to define the specificity and sensibility of GNMT as a biomarker in urine for liver damage, the presence of GNMT was tested in an active population. GNMT were positive in of the urine of patients with a liver damage, induced by poly-medication and by fat-burner consumption. These results provide the GNMT effectivity as non-invasive biomarker (Fig. 11A). Particularly, ALT and AST transaminases were negative in both GNMT-positive and negative patients, proving the early sensitivity of the marker one more time. In addition, GNMT expression was also determined by confocal microscopy and processing the urine sample by Cytospin^{™}, an innovative technique that provides a 3D protein proYection thought fluorescent microscope technologies and antibodies that stain the proteins. Here, the inventors observed similar results as those obtained before by Western Blot (Fig. 11B). Therefore, GNMT is positioned as a non-invasive routinely biomarker with the sensitivity to identify early liver damage in human samples. In addition, similarly as it happened in animal serum and urine samples, GNMT release can be also detected in urine through different approaches (such as WB or confocal microscopy) that confirm GNMT as a reliable non-invasive marker for liver disease. Moreover, the inventors found that the presence of glycine N-methyltransferase (GNMT) is increased in patients with liver injury and correlates with the fibrosis index. GNMT was determined by Western Blotting in a pre-validation of 40 urine sample belong to patients with liver pathology (Fig. 12A). The presence of the liver marker alanine aminotransferase (ALT) was not detected, whereas the presence of GNMT appeared to correlate with the grade of fibrosis, measured by Fibroscan (Fig. 12B).

### Example 8: GNMT in urine is a reliable biomarker for monitoring patients with liver damage.

As previously indicated, GNMT was a suitable non- invasive marker to follow up the liver pathology in preclinical animal models under treatment. Monitoring consumers of fat-burner slimming pills who had been previously determined to be GNMT positive three weeks after fat-burner intake disruption showed a significant decrease in urine GNMT levels, suggesting liver recovery as a consequence of stopping the intake of the slimming pills. (Fig. 13). In summary, GNMT appears as a reliable marker routinely non-invasive marker to characterise the reversion or progression of liver damage.

### Example 9: Development of an ELISA method for detecting GNMT in urine

ELISA test (a low-cost-feasible technique) was used to measure urine GNMT levels in 100 patients. These samples were compiled from an active population (general population with employment) with more than 50 clinical parameters. First, a positive correlation between GNMT detected by western blot and ELISA is shown in Fig. 14A and 14B). The previous results present another quantitative method, the ELISA, to detect GNMT release into urine in human samples. This method may be used to perform a diagnostic assay with GNMT as a reliable, non-invasive biomarker in urine for a wide range of liver damage from early to advance stage of liver damage. In addition, the presence of glycine N-methyltransferase (GNMT) was measured in a clinical cohort of patients (see table 2 below). 323 urines from well-characterised patients were analysed by homemade ELISA. Correlation between GNMT levels and Controlled attenuation parameter as indicator or steatosis (CAP_db/m) with 77% of sensibility. None of these patients showed high levels of transaminases ALT or AST.

**Table 2. ELISA analysis of GNMT in a clinical cohort of patients.**

| | | | GNMT Value | CAP_db/m |
|---|---|---|---|---|
| Spearman's Rho | GNMT Value | Correlation coefficient | 1 | ,137* |
| | | Sg. (bilateral) | 323 | ,014 |
| | | N | | 323 |
| | CAP_db/m | Correlation coefficient | ,137* | 1 |
| | | Sg. (bilateral) | ,014 | 323 |
| | | N | 323 | |

| | | | | |
|---|---|---|---|---|
| * 0,05 (bilateral) Significant correlation. | | | | |

### Example 10: GNMT is an age-independent liver damage biomarker and its presence in urine is stable

Importantly, chronic liver damage is associate to aging. In order to evaluate age-dependent changes in GNMT levels a general population that fluctuate from 65 to 85 years old was analysed by ELISA analysis, observing that GNMT is independent from the age (Fig. 15A). This data revealed that GNMT levels assess by ELISA test is not age-dependent and therefore GNMT biomarker is suitable for populations in all age ranges. As a conclusion, the capability to measure the levels of GNMT in urine describe this protein as a suitable non-invasive routine biomarker with a wide range of sensitivity, independent of the pathological etiology (viral interaction, drug, alcohol or toxins, NAFLD, NASH, autoimmune disorders, cholestatic disorders, metabolic disorders between others) and aging.

### Example 11: GNMT is enough to determine a non-invasive way to early stages of liver injury

In order to test if GNMT could be used as solo biomarker for liver damage and potentially exclude other known biomarkers (US8632982 B2), the inventors checked for the presence of published potential markers in urine. The inventors performed a three-phase screening of biomarkers to compare their suitability and sensitivity to be used as unique biomarker. Surprisingly, the inventors concluded that MAT1A, and BHM cannot be used as biomarkers (either as a joint marker of as a stand-alone marker) for liver damage, whereas GNMT offers a unique biomarker sensitivity to liver disease. As figure 16 shows, no other urine biomarker offers the same sensitivity or works in the same way as GNMT. These screening methods demonstrate that GNMT is suitable to detect from early to late stages of liver damage.

## Claims

1. An *in vitro* method for diagnosing liver disease in a subject and/or for triaging a subject for liver disease, wherein the method comprises:
a) determining the expression level of glycine N-methyltransferase (GNMT) in a urine sample from said subject and
b) comparing said expression level of GNMT with a reference value,
wherein an increased expression level of GNMT in said urine sample with respect to the reference value is indicative that the subject suffers from liver disease.

2. An *in vitro* method for monitoring progression of liver disease in a subject, wherein the method comprises:
a) determining the expression level of glycine N-methyltransferase (GNMT) in a urine sample from said subject at a first period of time;
b) determining the expression level of GNMT in a urine sample from said subject at a second period of time, wherein said second period of time is later than said first period of time; and
c) comparing the expression level of GNMT at the second period of time with the expression level of GNMT at the first period of time,
wherein an increased expression level of GNMT at the second period of time with respect to the expression level of GNMT at the first period of time is indicative that the liver disease is progressing in the subject;
wherein a decreased or an equal expression level of GNMT at the second period of time with respect to the expression level of GNMT at the first period of time is indicative that the liver disease is not progressing in the subject.

3. An *in vitro* method for monitoring the response to a therapy in a subject suffering from liver disease, wherein the method comprises:
a) determining the expression level of glycine N-methyltransferase (GNMT) in a urine sample from said subject prior to the administration of said therapy;
b) determining the expression level of GNMT in a urine sample from said subject during or after the administration of said therapy; and
c) comparing the expression level of GNMT during or after the administration of said therapy with the expression level of GNMT prior to the administration of said therapy,
wherein a decreased expression level of GNMT during or after the administration of said therapy with respect to the expression level of GNMT prior to the administration of said therapy is indicative that the subject has a positive response to the therapy;
wherein an increased or an equal expression level of GNMT during or after the administration of said therapy with respect to the expression level of GNMT prior to the administration of said therapy is indicative that the subject does not have a positive response to the therapy.

4. A method for selecting a subject to be treated for liver disease, wherein the method comprises:
a) determining the expression level of glycine N-methyltransferase (GNMT) in a urine sample from said subject, and
b) comparing said expression level of GNMT with a reference value,
wherein an increased expression level of GNMT in said urine sample with respect to the reference value is indicative that the subject is selected for treatment of an acute or a chronic liver disease.

5. The method according to any one of claims 1 to 4, wherein the expression levels of alanine aminotransferase (ALT) and/or aspartate aminotransferase (AST) in the urine sample from the subject are not increased with respect to a reference value.

6. The method according to any one of claims 1 to 5, wherein said liver disease is selected from liver injury, acute liver disease, and chronic liver disease.

7. The method according to any one of claims 1 to 5, wherein said liver disease is selected from drug-induced liver injury (DILI), non-alcoholic fatty liver disease (NAFLD), non-alcoholic steatohepatitis (NASH), liver fibrosis, steatosis, hepatitis, cirrhosis, veno-occlusive liver disease and Budd-Chiari syndrome.

8. The method according to claim 7, wherein said liver disease is selected from drug-induced liver injury (DILI), non-alcoholic fatty liver disease (NAFLD), non-alcoholic steatohepatitis (NASH), liver fibrosis, steatosis and cirrhosis.

9. The method according to any one of claims 1 to 8 wherein GNMT expression is determined by determining the activity level of the GNMT protein.

10. The method according to any one of claims 1 to 9 wherein GNMT expression is determined by determining the expression level of the GNMT protein.

11. The method according to claim 10 wherein GNMT expression level is determined by a method selected from the group consisting of Enzyme Linked Immunosorbent Assay (ELISA), Lateral Flow Assay (LFA), Point of Care Instrument (PoC), Western blot, immunoassay, microarray, mass spectrometry, surface plasmon resonance (SPR), Magnetic Resonance, fluorescent assays and Förster Resonance Energy Transfer (FRET).

12. Use of reagents specific for determining the expression level of glycine N-methyltransferase (GNMT) in a urine sample from a subject, for *in vitro* diagnosing a liver disease in said subject.
